(19)

Europäisches
Patentamt
European
Patent Office
Office européen
des brevets

(11) **EP 4 717 154 A1**

(12) **EUROPEAN PATENT APPLICATION**

(43) Date of publication:
**01.04.2026 Bulletin 2026/14**

(21) Application number: **24203659.8**

(22) Date of filing: **30.09.2024**

(51) International Patent Classification (IPC):
***A61B 5/00*** *(2006.01)*     ***A61B 5/026*** *(2006.01)*
***G01B 9/02091*** *(2022.01)*

(52) Cooperative Patent Classification (CPC):
**A61B 5/0066; A61B 5/0261; A61B 5/14553;**
**A61B 5/4064; G01B 9/02091**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Comind Technologies Limited**
**London EC1R 5EJ (GB)**

(72) Inventor: **BORYCKI, Dawid**
**London, EC1R 5EJ (GB)**

(74) Representative: **Mathys & Squire**
**The Shard**
**32 London Bridge Street**
**London SE1 9SG (GB)**

(54) **METHOD AND SYSTEM TO IMPROVE INTERFEROMETRY FOR TISSUE SENSING**

(57)     An aspect of the disclosure provides a interferometry apparatus comprising, an arrangement configured to provide a sample illuminating beam for interaction with a sample and subsequently to combine a received sample beam with a reference beam to provide interference in a combined beam; the arrangement comprising: a sample beam receiving portion for receiving the received sample beam after said interaction of the sample illuminating beam with the sample; a beam combiner configured to combine the received sample beam with the reference beam to generate the combined beam; and, an optical band-pass spatial filter arranged to filter the combined beam to provide a filtered beam; wherein the arrangement is further configured for providing the filtered beam to a detector configured to detect one or more intensities of the filtered beam.

FIG. 1

EP 4 717 154 A1

## Description

### Field of Invention

The present invention relates to methods and apparatus for interferometry of a sample, such as a biological sample, and more particularly to methods and apparatus for swept source interferometry of samples which may comprise living tissue, such as brain tissue.

### Background

Non-invasive measurements of cerebral blood flow (CBF) are challenging as direct access to the brain vasculature may be impossible and/or undesirable.

A number of optical methods of spectroscopy exist that have the potential to measure CBF and may be advantageous because they are non-invasive. Other physiological parameters such as intracranial pressure, ICP, and autoregulation function, ARF, are also of interest. Non-invasive measurement of such parameters is a significant technical problem.

Optical techniques provide some non-invasive methods. For example, photons may traverse the tissue of a sample and re-emerge after having been scattered down into the brain tissue. The greater the proportion of the collected photons that may have travelled down into the brain, the greater the sensitivity of suitable measurements to changes in blood flow in the brain.

When the light source is a coherent laser light, an index related to blood flow may be extracted from the collected light because photons of the collected light that travel along different paths through the sample may interfere with each other to generate spatial interference patterns. Such patterns may be known as speckle patterns, and may relate to how the photons were scattered on their path through the sample.

Changes in these speckle patterns over time may represent the movement of scatterers in the sample, which may be dominated by the movement of red blood cells in the microvasculature. By examining and suitably processing the changes over time in the collected light at processing unit, it may therefore be possible to infer changes in blood flow in the underlying (e.g., cerebral) tissue.

One method for measuring blood flow in such a manner is known as Diffuse Correlation Spectroscopy (DCS). In DCS, a coherent laser source (usually at near-infrared (NIR) or short-wave infrared (SWIR) wavelengths) may be coupled to the tissue of a sample, and light may be collected from a different point on the sample away from the source light probe. In DCS, the optical intensity (brightness) of either a single optical mode, or a small number of modes may be measured at high speed (usually ~ 100-500 kHz), and the autocorrelation of the resulting light intensity time-series may be used to determine an index of blood flow. The faster the measured signal decorrelates, the faster the blood flow in the brain tissue of sample may be.

Non-invasive measurements of other types of samples, including other types of tissues, remain a problem.

## Summary

Embodiments of the present disclosure may relate to interferometry systems and interferometry methods for performing measurements of samples.

Such methods may aim for the measurement of temporal variations in the interferometry signal to infer characteristics of the sample - such as the physiological parameters outlined above.

In such measurements, it can be a problem to achieve sufficient signal power and sufficient signal to noise ratio (SNR). One way to increase signal power in interferometric systems is to increase the reference beam intensity. However, this can cause saturation of the optical detector used to detect the interfered beam.

It is therefore a significant technical problem to increase signal to noise ratio and to preserve usable dynamic range of the optical detector.

Aspects and examples of the present disclosure aim to address the above technical problems and other related technical problems.

In an aspect there is provided an interferometry apparatus comprising an arrangement configured to provide a reference beam and a sample illuminating beam for interaction with a sample and subsequently to combine a received sample beam (e.g. the light scattered by the sample from the sample illuminating beam) with the reference beam to provide interference in a combined beam. The arrangement comprises an optical band-pass spatial filter arranged to filter the combined beam to provide a filtered beam. The arrangement may be configured to provide the filtered beam to an optical detector. The filtered beam may be configured for the detection of temporal variations in the filtered beam, for example it may be provided to a detector, where it may form an image on the detector. Such a detector may be controllable to obtain a timeseries of measurements of the filtered beam. Such interferometry may be performed with a wavelength tunable source of coherent light, such as a tunable laser. The apparatus may further comprise the wavelength tunable source of coherent light. The apparatus may further comprise the detector.

In an aspect there is provided an interferometry apparatus comprising,

an arrangement configured to provide a sample illuminating beam for interaction with a sample and subsequently to combine a received sample beam with a reference beam to provide interference in a combined beam;
the arrangement comprising:

a sample beam receiving portion for receiving the received sample beam after said interaction of the sample illuminating beam with the sample; a beam combiner configured to combine the received sample beam with the reference beam to generate the combined beam; and, an optical band-pass spatial filter arranged to filter the combined beam to provide a filtered beam; wherein the arrangement is further configured for providing the filtered beam to a detector configured to detect one or more intensities of the filtered beam.

The arrangement may comprise an optical arrangement, such as an arrangement of optical components configured to guide the respective beams of light along their respective paths.

The one or more intensities may comprise temporal variations. For example, the arrangement may be configured for the detection of temporal variations in the filtered beam.

The arrangement may be configured such that the filtered beam is suitable for the detection of temporal variations in intensity of the filtered beam. For example, the arrangement may be configured to form an image on a detector. Such images may enable temporal variations in at least one region of the beam to be detected. For example the image may be formed on a detector, which may be used to obtain a time series of measurement of the image from which these temporal variations can be detected. The variations may be detected in one or more spatially distinct regions of the image.

A interferometry apparatus comprising,

an arrangement configured to provide a sample illuminating beam for interaction with a sample and subsequently to combine a received sample beam with a reference beam to provide interference in a combined beam; the arrangement comprising:

a sample beam receiving portion for receiving the received sample beam after said interaction of the sample illuminating beam with the sample; a beam combiner configured to combine the received sample beam with the reference beam to generate the combined beam; and, an optical band-pass spatial filter arranged to filter the combined beam to provide a filtered beam, wherein the spatial filter comprises an optical element operable to receive said combined light beam and convert the combined light beam into a spatial frequency representation of the combined light beam, and the spatial filter comprises a frequency-selective attenuating component configured to attenuate selected portions of the frequency representation of the combined light beam to provide the band-pass spatial filter.

The band-pass spatial filter may be a high-pass spatial filter.

The arrangement may be further configured for providing the filtered beam to a detector configured to detect one or more intensities of the filtered beam.

The interferometry apparatus may further comprise the detector.

The optical band-pass spatial filter may be a high-pass spatial filter.

The interferometry apparatus may further comprise the detector, which may also be made and sold separately. The detector may comprise a multipixel detector such as an image sensor - e.g. a sensor array. Accordingly, by using these or other types of image detector, an image formed on the detector may be suitable for the detection, from a series of such images, temporal variations in a plurality of spatially distinct regions of the image.

The arrangement may also be used with a tunable laser, or other source of coherent light, operable to provide a wavelength swept light signal. The arrangement may be provided as an optical train - for example, the optical train may comprise an assembly of optical components configured to guide a beam of light along selected paths, such as from a source of coherent light to a detector.

It will be appreciated in the context of the present disclosure that the light source and the detector may be made and sold separately. Accordingly, the arrangement may also be made and sold separately - e.g., as an optical train configured to perform the functions ascribed to the arrangement as described and/or claimed herein. Such an arrangement may comprise lenses and optical fibres and other components but the need for and disposition of such elements will vary from case to case and in some arrangements, these things may be dispensed with altogether.

The arrangement may be configured to use the filtered combined beam to form an image on the detector. The detector may be configured to provide signals to a data processor to enable the data processor to detect the temporal variations in a plurality of spatially distinct regions of the image. Accordingly, the detector may comprise an image sensor having an array of sensor pixels or any other appropriate type of detector.

Providing interference in the combined beam may also be referred to as interfering the received sample beam with the reference beam to form the combined beam. Typically, this means that the arrangement is configured so that the optical path difference between the reference beam and the beam which interacts with the sample may be less than the coherence length of the light source.

The apparatus may comprise a sample probe arrangement configured to be fixed to a sample, such as biological tissue, to provide optical coupling of the sample illuminating beam to the sample and optical coupling of the received sample beam from the sample.

The sample probe arrangement may comprise a sample delivery probe, configured to deliver light to the sample. The light delivered to the sample is referred to herein as the sample illuminating beam. The sample probe arrangement may also comprise a sample receiving probe, configured to receive the scattered light from the sample (referred to herein as the received sample beam). The sample delivery probe and the sample receiving probe may be separate from each other.

The sample delivery probe may comprise a collimating lens (L1).

The arrangement may comprise a sample beam receiving portion, in which the received sample beam is coupled to the arrangement from the sample receiving probe. A multi-mode fiber (MMF) or a plurality of single mode fibers (SMF) may couple the sample receiving probe to the arrangement.

The apparatus may be configured for swept source interferometry. It will be appreciated in the context of the present disclosure that the optical spatial bandpass filter generally comprises a spatial filter which operates in the optical domain (as opposed to the digital domain) to attenuate spatial frequencies outside a passband of the spatial filter (e.g. to attenuate frequencies in a stop-band more than those in the pass-band. One example of a band-pass filter is a high-pass filter.

The spatial filter may comprise a first optical element, such as a focussing element, operable to receive the combined beam and separate the combined beam into portions, each portion comprising a different spatial frequency component of the combined beam. The spatial filter may also comprise a frequency-selective attenuating component configured to attenuate selected portions of the frequency representation beam.

The first optical element may comprise any focussing element such as at least one of a refractive lens, a zone-plate, and a grating.

The frequency-selective attenuating component may be configured to attenuate the selected portions more than other portions of the frequency representation beam to provide the spatial filter. For example, the frequency-selective attenuating component may block the selected portions.

A second optical element (L5) may be configured to recombine the frequency-representation beam, received from the frequency-selective attenuating component, to provide the filtered beam.

The frequency-selective attenuating component may comprise a non-transparent region, which may be disposed in a transparent carrier. The non-transparent region may be substantially aligned with a center axis of the frequency-representation beam.

Said non-transparent region may be configured to attenuate at least a DC spatial frequency component of said frequency-representation beam. Said non-transparent region may be one of a circular disc, an oval disc, a rectangle, and a cross-shape.

The apparatus may further comprise a light source configured to provide light to a first beam splitter for providing the sample illuminating beam and the reference beam, for example wherein the light source is a tunable laser.

Said tunable laser may be configured to tune wavelength in a sweep pattern.

The apparatus may comprise a reference beam attenuator disposed between said first beam splitter and said second beam splitter. The reference beam attenuator may be controllable to adjustably attenuate the reference beam.

The apparatus may comprise a data processor configured to control the reference beam attenuator based on an indication of light detected by the detector. The data processor may be configured to determine the indication.

The indication may provide a performance measure, such as dynamic range or signal-to-noise ratio, for example wherein the controller is configured to control the attenuator so as to increase a performance measure. Accordingly, it can be seen that the indication provides a feedback signal for control of the reference beam attenuator.

The optical detector may comprise a plurality of sensor elements. The sensor elements may be arranged in an array, such as in an image sensor. For example the sensor elements may be provided by sensor pixels of an image sensor. The sensor elements may be provided by photodetectors.

The sensor array may be one of:

(a) an array of unbalanced or balanced photodetectors;
(b) a CMOS sensor array
(c) an InGaAs sensor array
(d) a CCD sensor array; or
(e) an Avalanche Photodiode sensor array.

The apparatus may comprise a bundle of SMF, wherein each SMF is arranged to provide a portion of the filtered beam to a respective corresponding sensor element, such as an element of the sensor array.

The apparatus may comprise a data processor, coupled to the optical detector.

The sample may be a part of a subject's head, such as the scalp or brain, and the controller is configured to determine, based on said temporal variations of the filtered beam, intracranial pressure, ICP, cerebral blood flow, CBF, or auto regulation function, ARF.

The apparatus may comprise an envelope determiner arranged to provide an envelope signal based on said temporal variations.

The envelope determiner may comprise a frequency shifter, arranged to receive said filtered beam and to determine an envelope of the filtered beam.

An aspect of the invention provides a method of performing interferometry to detect spatially distributed time varying interference effects in an interferometric image, the method comprising:

providing a sample illuminating beam to a sample, combining, with a reference beam, a received sample beam, to provide a combined beam;
spatially filtering the combined beam with an optical spatial filter to provide spatial band-pass filtering a filtered combined beam; and
using the filtered combined beam to form an image on a detector, the detector being configured to detect temporal variations in a plurality of spatially distinct regions of the image.

It will be appreciated in the context of the present disclosure that the received sample beam may be provided by the sample illuminating beam interacting with (e.g., being scattered by) the sample. It will also be appreciated that the "beams" referred to herein are beams of light.

The method may comprise providing a wavelength swept light signal to a first beam splitter to provide the sample illuminating beam and the reference beam, for example wherein the wavelength swept light is provided by a tunable laser.

The method may comprise controlling a reference beam attenuator to adjustably attenuate the reference beam before combining the reference beam with the received sample beam.

The method may comprise controlling the reference beam attenuator based on a feedback signal generated by said detector.

The reference beam attenuator may be controlled based on the feedback signal to increase a performance measure, such as dynamic range or signal-to-noise ratio.

The sample may be a part of a subject's body, such as tissue having blood flow. For example, the sample may comprise a part of the head and the method may comprise determining, based on said temporal variations, at least one of intracranial pressure, ICP, cerebral blood flow, CBF, or autoregulation function ARF.

The method may comprise determining an envelope of said temporal variations.

The envelope of said temporal variations may be determined by providing the filtered beam to a frequency shifter, arranged to receive said filtered beam and to determine the envelope of the filtered beam.

An embodiment may provide an interferometric near-infrared spectroscopy (iNIRS) system comprising any one or more of the interferometry systems described or claimed herein. In an iNIRS system, a light source may provide source light to the sample and to a reference path which then interferes with the collected light from the sample.

iNIRS may have several advantages over DCS.

For example, iNIRS may be able to resolve the time-of-flight of light through sample. iNIRS may also provide optical homodyne gain to improve the quality of the measured signal, and may better reject environmental light interference.

These advantages may be significant, because they may enable improved depth sensitivity and specificity, and may provide absolute instead of relative measures of the optical properties of sample.

Embodiments of the disclosure comprise physiological measurement apparatus comprising any of the interferometry apparatus or systems described or claimed herein. Such apparatus typically comprises a sample probe, coupled to provide light to the sample beam receiving portion. Such apparatus and/or probes may be adapted for use in a clinical environment, such as a sterile field.

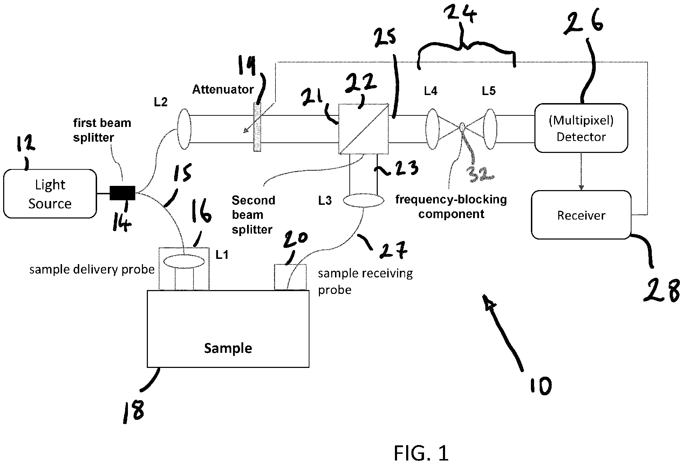

An aspect of the disclosure provides an interferometry system, such as that described below with reference to Figure 1. Such a system may comprise:

a light source for generating processing light;
a first beam splitter, splitting said processing light into a sample light and a reference light (e.g., the sample beam and reference beam as described herein);
a sample delivery probe, receiving and directing said sample light into a sample;
a sample receiving probe receiving a portion of said sample light when it has travelled through said sample;
a second beam splitter receiving and combining said reference light and said received portion of said sample light into a combined beam;
a lens (L4), said lens operable to receive said combined beam and generate a frequency-representation beam;
a frequency-blocking component receiving said frequency-representation beam and blocking a portion of said frequency-representation beam and passing another portion of the beam, generating a filtered beam.

A second lens (L5), said lens operable to receive said filtered beam and generate a time-representation beam;

a detector receiving said time-representation beam, generating an electrical output signal based on said received time-representation beam; and,
a receiver operable to process said electrical output signal.

The frequency-blocking component may comprise a transparent carrier carrying a non-transparent shape. The non-transparent shape may be substantially aligned with a centre axis of said received frequency-representation beam.

The non-transparent shape may be a disc positioned to block at least a DC spatial frequency component of said frequency-representation beam.

The non-transparent shape may be one of a circular disc, an oval disc, a rectangle, and a cross-shape.

The light source may be a tunable laser.

Any feature of any one of the examples disclosed herein may be combined with any feature of other such examples. Method embodiments may be implemented in suitably configured hardware, for example, the present disclosure includes apparatus providing means for implementing each of the method steps described and/or claimed herein.

The configuration of the specific hardware described herein may be employed in methods implemented using other hardware.

## Brief Description of Drawings

Embodiments of the disclosure will now be described in detail with reference to the accompanying drawings, in which:

Figure 1 shows a swept source interferometry apparatus according to the present disclosure;
Figure 2 illustrates the light signals at different stages in an interferometry apparatus such as that illustrated in Figure 1;
Figure 3 shows a series of three plots of a combined beam and the effect of spatial filtering as provided in embodiments of the present disclosure; and
Figure 4 provides a diagram of a frequency attenuating element such as may be used in some spatial filters according to the present disclosure.

In the drawings like reference numerals are used to indicate like elements.

## Specific Description

Figure 1 shows an interferometry apparatus 10 comprising a detector 26, a data processor 28, a light source 12, a sample probe 16, 20, and an arrangement of optical components 14, 19, 21, 22, 23, 24, 25 configured to guide light along specific paths from the light source 12 to the detector 26.

This arrangement 14, 19, 21, 22, 23, 24, 25 illustrated in Figure 1 provides two such paths for light.

This arrangement comprises a first beam splitter 14, a beam combiner 21, 22, 23, 25 and a spatial filter 24. The arrangement also comprises a controllable attenuator 19, but this is optional. A number of optic fibres and/or lenses L1, L2, L3, L4, L5 may also be provided and their disposition may vary depending on the characteristics and spatial arrangement of the components of the arrangement.

The first beam splitter 14 is arranged to receive a beam of light from the light source 12, to split that beam of light into a first portion which it directs along a first path and a second portion which it directs along a second path. The first path goes via the sample 18, and the part of the beam which travels this path is referred to herein as comprising the sample illuminating beam and the received sample beam (the sample illuminating beam after it has interacted with the sample). The second path does not go via the sample 18 and is used to provide a reference for interferometry and so is referred to a reference beam.

The sample probe arrangement 16, 20 comprises a sample delivery probe 16 for delivering the sample beam to a region of a sample 18, and a sample receiving probe 20 for receiving light back from the sample 18 after it has interacted with the sample 18.

The first beam splitter 14 is connected to the sample delivery probe 16 by an optic fibre 15 to carry the sample beam to the sample delivery probe 16. The sample delivery probe 16 comprises an aperture arrangeable for optical coupling of the sample beam with a surface of the sample 18. The sample receiving probe 20 also comprises such an aperture.

The sample delivery probe 16 may comprise a collimating lens L1 or other optical element configured to provide light from the optic fibre 15 toward the sample 18. In the arrangement illustrated in Figure 1, where the apparatus is in use, the sample 18 provides a degree of optical coupling between the sample delivery probe 16 and the sample receiving probe 20.

The beam combiner 21, 22, 23, 25 may comprise a second beam splitter 22 (such as a half-silvered mirror or similar optical element) and has two inputs 21, 23 and an output, 25. The first input 21 is positioned on the second path of the arrangement, to receive the reference beam from the first beam splitter 14. For example, this first input 21 may be connected to the first beam splitter 14 by an optic fibre and/or lens arrangement. As illustrated in Figure 1, the controllable attenuator 19 is also in the first light path, interposed between the first beam splitter 14 and the first input 21 of the beam combiner, e.g., for providing the reference beam to the first input 21 of the beam combiner.

The second input 23 of the beam combiner is configured and arranged for receiving the received sample beam from the sample receiving probe 20. For example, the sample receiving probe 20 may be connected to the second input of the beam combiner via a further optic fibre 27, which may be a multimode fibre (MMF) or a plurality of single mode fibres, which may be arranged together such as in a bundle, for example. This may serve as a sample beam receiving portion of the arrangement, but other means of coupling the sample beam into the beam combiner may be used.

Accordingly, the second beam splitter 22 may be arranged to receive the reference beam from the first input 21 of the beam combiner and the received sample beam from the second input 23 and to superpose the two beams received at the inputs to provide a combined beam to the output 25 of the beam combiner 22.

A spatial filter 24 is provided in the arrangement between the output 25 of the beam combiner and the detector 26. The spatial filter 24 comprises optical components arranged to provide a pass-band and an attenuated-band in a filtered beam. The attenuated-band may comprise lower frequencies than the pass-band - for example to provide a high-pass spatial filter. In some embodiments, the spatial filter may also be arranged to attenuate selected higher spatial frequencies, so that frequency components below a first cut-off frequency are attenuated (e.g. blocked) and frequency components above a second cut-off frequency are also attenuated. The pass-band of the filter being between the first cut-off frequency and the second cut-off-frequency. The second-cut off is optional, so the pass band may simply comprise all frequencies above the first cut-off. One implementation of such a high-pass spatial filter is described below. A first and second cut-off frequency may be defined by some specific attenuation level that may be achieved at a given frequency when comparing the input and the output beams to spatial filter 24, for example.

The detector 26 may comprise a sensor array, such as an image sensor, arranged so that the filtered beam can form an image on the detector 26. The detector is configured to provide electrical signals, indicative of said image, to the data processor 28.

The controllable attenuator 19 may also be connected to the data processor 28 to enable the data processor 28 to control the degree of attenuation it applies to the reference beam. The degree of attenuation may be between zero and a selected maximum attenuation depth, such as a selected proportion of the incident light intensity.

The light source 12 generally comprises a controllable source of coherent light, such as a laser. Examples of lasers which are particularly useful in the present disclosure include tuneable lasers, able to provide a wavelength tuned signal which may provide a sweep of wavelengths. It will be appreciated in the context of the present disclosure that the arrangement may be arranged so that the optical path length of the first path from the light source to the detector matches the optical path length of the second path (e.g., optical path length via the sample may be configured to match the optical path length of the reference beam). For example, any difference between the two optical path lengths may be less than the coherence length of the light source.

In operation, the light source 12 generates a laser beam having a swept frequency - for example a deterministic, possibly periodic, frequency-sweep pattern. Thus, the frequency of the source light beam may vary with time. The first beam splitter 14 splits this beam and provides a first part of the beam (the sample illuminating beam) to the sample delivery probe 16. The sample delivery probe 16 provides the sample illuminating beam into the sample 18. This results in light being provided, via the sample 18, to the sample receiving probe 20. This received light is referred to herein as the received sample beam. The received sample beam, having interacted with the sample 18, is then provided from the sample receiving probe 20 to the second input 23 of the beam combiner 21, 22, 23, 25.

The reference beam, provided by the other part of the source light beam, is also provided to the beam combiner 21, 22, 23, 25 at its first input 21. The beam combiner superposes the reference beam and the sample beam so as to cause the two beams to interfere.

For example, if the source light (and the reference beam) may be written as:

$$x(t,f) = cos(2*pi*f(t)*t),$$

where t is time and f(t) is the frequency as a function of time due to the tuning of the laser. The sample beam may be delayed, due to its interaction with the sample, by a delay time $dT$. In which case the sample beam may be written as $x(t+dT)$. It will be appreciated in the context of the present disclosure that the light wave which constitutes the laser beam may not be a pure sinusoid, but the present example is provided for illustration of a principle of operation of the apparatus. It will further be appreciated that there are many paths though the sample, so the indication above is merely illustrative and the actual received sample beam comprises the sum of differently attenuated paths through the sample. The discussion below refers to one path by way of illustrative example. The actual received sample beam contains many such signals.

The superposition, in the beam combiner of the reference beam x(t) and the received sample beam $x(t+dT)$ provides a resulting signal having a signal envelope which is proportional to $cos(2*pi*[f(t)-f(t+dT)]t)$. In other words, the combined beam is modulated with a beat frequency (or beat frequencies, plural) arising from path length difference(s) caused by the transit of the received sample beam through the sample 18.

The instantaneous frequency difference between the reference beam and the received sample beam indicates the path delay that a photon through the sample experienced, as compared to the reference beam. On average, the deeper a photon travels, the longer its optical path length and thus delay as compared to the reference beam. Longer paths also generally lead to stronger light attenuation in the sample 18. The frequency sweep of the light means that this delay results in a frequency difference which manifests as "beats" in the intensity of the interfering beams. Different spatial locations in the sample beam may thus exhibit different path length differences and hence different "beat" frequencies.

By sweeping the wavelength of the sample illuminating beam, temporal variations may be detected in pixels of the interferometry image formed, at the detector 26, by the combined beam. The local beat frequency (or beat frequencies) present in each pixel can be detected. The data processor can then infer penetration depth information from the beat frequency (or beat frequencies).

The detector 26 may provide, to the data processor 28, an electrical signal (such as a digital signal) representing the image. A series of such signals may be provided, each one comprising an image frame obtained from the sensor array.

The data processor 28 may be configured to determine, based on such signals an indication of a performance measure of the system. The performance measure may be based on an image or the series of images or on a time series extracted from such images or one or more pixels of the images. Examples of the performance measure include a signal to noise ratio, a signal power measure such as total signal power or average signal power, an intensity range, a histogram measure, correlation measures, or similar. The data processor 28 may use the performance measure to determine a feedback signal and use the feedback signal to control the attenuator 19 so as to improve the performance measure.

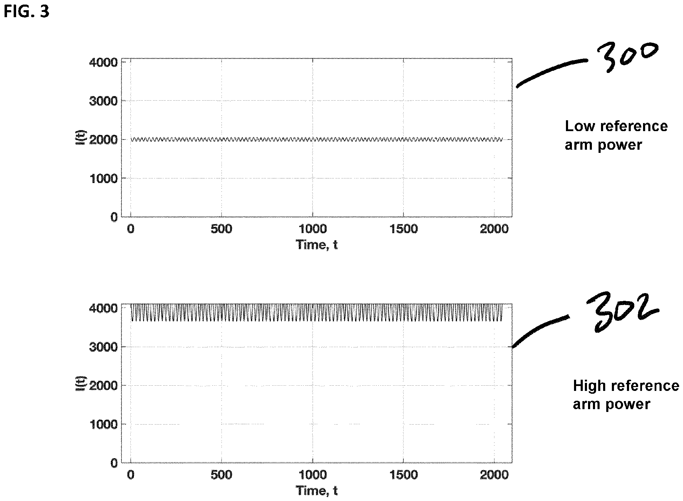

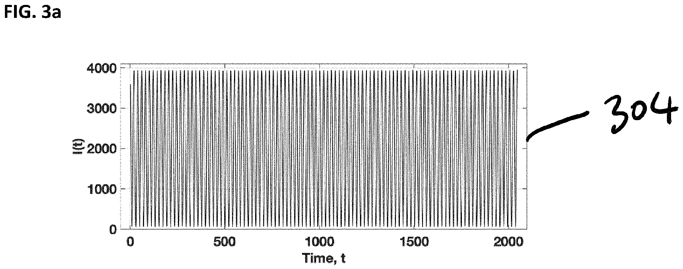

The temporal variations in the image at the detector 26 may be localised (e.g. the information may be conveyed in higher spatial frequencies of the optical signal). In addition, relative to the total signal power in the combined beam, the temporal variations may be relatively low power. This may create a problem in that the detector 26 may be saturated by the total power in the combined beam and/or the signal-level resolution (as opposed to spatial resolution) of the sensor may be compromised in order to achieve sufficient dynamic range to accommodate that high signal power. The problem of saturation may be particularly significant when the reference beam power at input 21 of beam combiner 22 may be large. This problem is illustrated in Figure 3, in which the top graph 300 shows a time-varying (AC) combined signal component comprising a large DC component that is due to the reference beam. It is possible to increase the total power (including the AC component) of the combined beam by increasing the intensity of the reference light beam (see second plot 302 in Figure 3) but the high signal total power may make it more likely that the detector saturates or "clips" and useful signal information is lost.

The present disclosure may address this problem by use of the spatial filter 24 to reduce total signal intensity reaching the detector 26 while preserving the localised time varying signals which may encode penetration depth information in the combined beam image. This may result in a greater amount of the dynamic range at any given pixel being available for the measurement of temporal variations in the intensity at that pixel, as illustrated in plot 304 in Figure 3a.

A variety of different implementations of the disclosure above will be apparent to the skilled addressee in the context of the present disclosure.

For example, there may be a variety of implementations of a spatial filter 24. One such implementation will now be described. In the apparatus of Figure 1, the spatial filter may comprise a first lens (L4) which is arranged to receive the combined beam from the beam combiner, and to focus the beam at a focal plane behind the first lens (L4). The spatial filter also comprises a frequency-selective attenuating element 32 and a second lens (L5).

The frequency-selective attenuating element 32 is disposed at the focal plane of the first lens L4 and arranged to attenuate selected portions of the beam at the focal plane. Typically, the frequency-selective attenuating element comprises non-transparent portions held in a transparent carrier and positioned to block or reduce the intensity of light of certain parts of the beam cross-section. The non-transparent portions may be centred on the optic axis of the elements L4 and L5 (which are generally coincident with the beam axis). It will be appreciated in the context of the present disclosure that, at the focal plane of the lens, the 2D distribution of light across the beam corresponds to the spatial frequency content of the light incident on the lens - e.g., the 2D Fourier transform of the incident beam. This is where the frequency-selective attenuating element 32 may be placed. Low spatial frequencies are disposed near the optical axis and higher spatial frequencies further away. The non-transparent portions of the frequency-selective attenuating element 32 thus offer one way to attenuate selected spatial frequencies. Any desired filter transfer function may be provided by the disposition of these non-transparent portions in the transparent carrier.

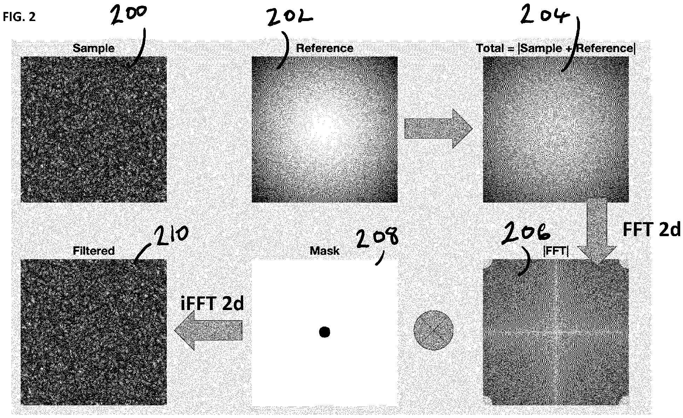

As illustrated in Figure 2, the reference beam 202 and sample beam 200 may each have a given intensity profile across the beam. The combined beam 204 present at the output 25 of the beam combiner is the superposition of these two intensity profiles as generated by any interference effects between the two beams. The combined beam 204 may thus have an intensity profile $g(x,y)$, where g is the local intensity and x and y are cartesian coordinates transverse to the beam. At a distance of one focal length f after the first lens L4, the intensity profile is $G(k_x, k_y)$, where G is the Fourier transform of g and $k_x$ and $k_y$ are the spatial frequencies in x and y respectively. This is illustrated in the fourth plot 206 of Figure 2. It can be seen in Figure 2 that the low frequency parts of the beam carry the most intensity, because the reference beam is substantially uniform (or at least has a profile matching the source). This appears in plot 206 of Figure 2 as a "cross-hair" type image because the lowest frequencies are in the centre of the picture.

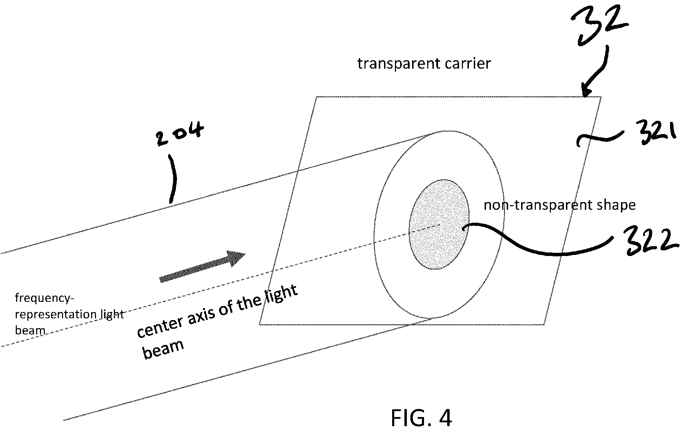

The frequency-selective attenuating element 32 filters the combined beam 204 by attenuating selected parts of the beam 206 at the focal plane $G(k_x, k_y)$ while allowing other parts to pass with less attenuation. For example, some parts may be blocked by non-transparent portions, such as opaque portions of the attenuator. This may be done by a frequency-blocking component placed at the focal plane of the first lens (L4) of the spatial filter 32. This component may achieve frequency blocking by physically masking certain parts of the beam at the focal plane of the lens L4 as in the plot labelled "Mask" 208. Figure 4 illustrates an example in which the frequency-selective attenuating element 32 comprises a transparent carrier 321 supporting non-transparent portions 322. In this example, the non-transparent portions 322 provide a mask in the form of an opaque (e.g., black) disc on an otherwise transparent carrier 321. The carrier 321 may be made of glass, for example. This attenuates the most significant low-frequency components, and in particular the DC offset. A second lens L5 may be positioned so that the frequency-selective attenuating element (and the focal plane of the first lens) are at the object focus of the second lens L5. For example, if the two lenses have the same focal length, $l_f$, the attenuating element would be spaced by $l_f$ from the first lens and the second lens would be spaced by f from the attenuating element.

This second lens L5, in effect inverts the Fourier transform performed by the first lens L4. The detector 26 may be disposed after the second lens L5), to receive the filtered beam (see "Filtered", 210 in Figure 2).

The spatial filter need not be implemented in this way and other implementations are contemplated. Examples may include zone plates and diffraction gratings. Any appropriate optical spatial filter may be used.

Other variations are also contemplated.

Typically, the light source is a laser such as a tunable (variable wavelength) wavelength laser, but other sources of coherent light may be used.

The first beam splitter 14 may be configured to provide a greater amount of light intensity into the first path of the arrangement (the sample beam) than is provided to the second path (reference beam). In one example the power split may be 15% to the reference beam and 85% to the sample beam.

The sample delivery probe 16 may be provided by the end of an optic fibre (or a bundle of optic fibres), which may be removably fixed to the sample.

The sample 18 may be a human head, comprising a brain.

The aperture for optical coupling between the sample delivery probe and the sample may be provided simply by the end of the optic fibre or by an optical assembly comprising one or more lenses. For example, such an optical assembly may be configured to spread the sample beam to generate a beam with a larger diameter. This may increase the total usable beam power.

The aperture of the sample receiving probe 20 may also be provided by an end of an optic fibre or optic fibre bundle. It may also comprise lenses, for example arranged to collect light from an area of the sample and collimate it into a beam (or optic fibre) of narrower diameter than the area from which it was collected.

The sample probes 16, 20 may comprise one or a plurality of fibres, a multi-mode fiber (MMF), or a bundle of SMF comprising any number of single mode fibers (SMF).

The apparatus shown in Figure 1 comprises lenses, but these are optional and may be provided by one or more other optical components or assemblies of such components. The function these lenses provide may be provided by other equivalent elements or their function may be integrated into other components.

The non-transparent parts of the attenuating element in the spatial filter may have any appropriate shape to provide spatial filtering / attenuating.

The frequency-sweep of the light source may be linear. For example, it may comprise a series of linear ramps, which may be arranged in a saw tooth or triangular waveform. The wavelength range of each sweep may be small in comparison to the wavelength of NIR, for example it may be less than 10nm, for example less than 5nm, for example about 1 nm.

The filtered beam could be carried to the sensor array of the detector by an MMF, or by an SMF bundle. In such a bundle each SMF may be coupled to a pixel of a sensor or to a single channel detector in a bank of detectors.

It will be appreciated in the context of the present disclosure that the "sample illuminating beam" and the "received sample beam" are different parts of the same light beam. The sample illuminating beam refers to the light beam before it interacts with the sample and the received sample beam refers to that light after it has been scattered by (e.g., interacted with) the sample.

Embodiments of the disclosure may be used in measurement of IntraCranial Pressure (ICP), Cerebral Blood Flow (CBF) and Autoregulation Function (ARF) which is a cerebral autoregulation measure.

Other embodiments are envisaged.

It will be appreciated from the discussion above that the embodiments shown in the Figures are merely exemplary, and include features which may be generalised, removed or replaced as described herein and as set out in the claims. With reference to the drawings in general, it will be appreciated that schematic functional block diagrams are used to indicate functionality of systems and apparatus described herein.

It will be appreciated however that the functionality need not be divided in this way, and should not be taken to imply any particular structure of hardware other than that described and claimed below. The function of one or more of the elements shown in the drawings may be further subdivided, and/or distributed throughout apparatus of the disclosure. In some embodiments the function of one or more elements shown in the drawings may be integrated into a single functional unit.

In some examples the functionality of the data processor described herein may be provided by a general purpose processor, which may be configured to perform a method according to any one of those described herein. In some examples the data processor may comprise digital logic, such as field programmable gate arrays, FPGA, application specific integrated circuits, ASIC, a digital signal processor, DSP, or by any other appropriate hardware. In some examples, one or more memory elements can store data and/or program instructions used to implement the operations described herein. Embodiments of the disclosure provide tangible, non-transitory storage media comprising program instructions operable to program a processor to perform any one or more of the methods described and/or claimed herein and/or to provide data processing apparatus as described and/or claimed herein. The controller may comprise an analogue control circuit which provides at least a part of this control functionality. An embodiment provides an analogue control circuit configured to perform any one or more of the methods described herein.

The above embodiments are to be understood as illustrative examples. Further embodiments are envisaged. It is to be understood that any feature described in relation to any one embodiment may be used alone, or in combination with other features described, and may also be used in combination with one or more features of any other of the embodiments, or any combination of any other of the embodiments.

Furthermore, equivalents and modifications not described above may also be employed without departing from the scope of the invention, which is defined in the accompanying claims.

**Claims**

1. A interferometry apparatus comprising,

    an arrangement configured to provide a sample illuminating beam for interaction with a sample and subsequently to combine a received sample beam with a reference beam to provide interference in a combined beam;
    the arrangement comprising:

    a sample beam receiving portion for receiving the received sample beam after said interaction of the sample illuminating beam with the sample;
    a beam combiner configured to combine the received sample beam with the reference beam to generate the combined beam; and, an optical band-pass spatial filter arranged to filter the combined beam to provide a filtered beam;
    wherein the arrangement is further configured for providing the filtered beam to a detector configured to detect one or more intensities of the filtered beam.

2. The apparatus of claim 1 wherein the optical train is configured to use the filtered beam to form an image on the detector for the detection of the temporal variations in a plurality of spatially distinct regions of the image.

3. The apparatus of claim 1 or 2 further comprising a light source configured to provide light to a first beam splitter for providing the sample beam and the reference beam, for example wherein the light source is a tunable laser.

4. The apparatus of claim 3 wherein said tunable laser is configured to tune wavelength in a sweep pattern.

5. The apparatus of any preceding claim comprising a reference beam attenuator disposed between the first beam splitter and the beam combiner, wherein the reference beam attenuator is controllable to adjustably attenuate the reference beam.

6. The apparatus of claim 5 comprising a data processor configured to control the reference beam attenuator based on an indication of light detected by the detector.

7. The apparatus of claim 6 wherein the indication provides a performance measure, such as dynamic range or signal-to-noise ratio, for example wherein the controller is configured to control the attenuator so as to increase a performance measure.

8. The apparatus of any preceding claim comprising a sample probe configured to be fixed to a sample to provide optical coupling of the sample beam with the sample.

9. The apparatus of claim 8 wherein the sample probe comprises a sample delivery probe, configured to deliver the sample beam to the sample and a sample receiving probe, configured to receive the received sample beam from the sample, for example wherein said sample receiving probe couples the received sample beam to the arrangement via a multi-mode fiber (MMF) or a single mode fiber (SMF).

10. The apparatus of any preceding claim, wherein the spatial filter comprises an optical element operable to receive said combined light beam and convert the combined light beam into a frequency-representation of the combined light beam comprising separated spatial portions, each spatial portion comprising different spatial frequency components of the combined light beam,
    for example wherein the spatial filter comprises a frequency-selective attenuating component configured to attenuate selected portions of the frequency representation of the combined light beam.

11. The apparatus of any preceding claim wherein the detector comprises a plurality of sensor elements, for example wherein the sensor elements are arranged in an array, for example one of:

(a) an array of unbalanced or balanced photodetectors;
(b) a CMOS sensor array
(c) an InGaAs sensor array
(d) a CCD sensor array; or
(e) an Avalanche Photodiode sensor array.

12. The apparatus of any preceding claim comprising a bundle of single mode fibres SMF, wherein each SMF is arranged to provide a portion of the filtered beam to a respective corresponding one of a plurality of sensor elements.

13. The apparatus of claim 12 wherein each of the plurality of sensor elements is provided by: (a) a corresponding one of a plurality of photodetectors; or (b) at least one sensor pixel of a detector, such as an image sensor.

14. The apparatus of any preceding claim comprising a data processor, coupled to the detector, for example wherein the sample is a biological tissue, for example an *in vivo* biological tissue such as a part of a living subject's head and the controller is configured to determine at least one of: blood pressure, intracranial pressure, ICP, cerebral blood flow, CBF, or autoregulation function, ARF, based on said detector signal.

15. A method of performing interferometry to detect spatially distinct time varying interference effects in an interferometric image, the method comprising:

providing a sample beam to a sample,
combining with a reference beam with the sample beam from the sample to provide a combined beam;
applying a band-pass spatial filter to the combined beam; and
using the result of said filtering to form a series of images; and
detecting spatial or temporal variations in a plurality of spatially distinct regions of the image.

FIG. 1

FIG. 2

FIG. 3

_300_

Low reference
arm power

_302_

High reference
arm power

**FIG. 3a**

304

transparent carrier

*32*

*321*

*204*

non-transparent shape

*322*

frequency-
representation light
beam

center axis of the light
beam

FIG. 4

Europäisches
Patentamt
European
Patent Office
Office européen
des brevets

## EUROPEAN SEARCH REPORT

**Application Number**

EP 24 20 3659

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/113439 A1 (MOHSENI HOOMAN [US]) 16 April 2020 (2020-04-16) | 1-6,8, 11,14,15 | INV. A61B5/00 |
| Y | * paragraphs [0010], [0066], [0121], [0129] - [0131], [0073] - [0086], [0096] - [0098], [0064] - [0069], [0224] - [0231] * <br> * figure 22 * | 9,10,12, 13 | A61B5/026 G01B9/02091 |
| X | US 2002/099295 A1 (GIL AMIR [IL] ET AL) 25 July 2002 (2002-07-25) * paragraphs [0064] - [0069], [0224] - [0231], [0276], [0309], [0310] * | 1,5-7,15 | |
| Y | GB 2 621 991 A (COMIND TECH LIMITED [GB]) 6 March 2024 (2024-03-06) * page 5, lines 29-38 * | 9,10,12, 13 | |

| | |
|---|---|
| | **TECHNICAL FIELDS SEARCHED (IPC)** |
| | A61B <br> G01B |

The present search report has been drawn up for all claims

| Place of search | Date of completion of the search | Examiner |
|---|---|---|
| The Hague | 20 February 2025 | Kowalczyk, Szczepan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 20 3659

This annex lists the patent family members relating to the patent documents cited in  the above-mentioned European search report.
The members are as contained in the European Patent Office EDP file on
The European Patent Office is in no way liable for these particulars which are merely given for the purpose of information.

20-02-2025

| Patent document cited in search report | | Publication date | Patent family member(s) | | Publication date |
|---|---|---|---|---|---|
| US 2020113439 | A1 | 16-04-2020 | NONE | | |
| US 2002099295 | A1 | 25-07-2002 | AU | 1547101 A | 04-06-2001 |
| | | | US | 2002099295 A1 | 25-07-2002 |
| | | | WO | 0137717 A2 | 31-05-2001 |
| GB 2621991 | A | 06-03-2024 | GB | 2621991 A | 06-03-2024 |
| | | | WO | 2024042338 A1 | 29-02-2024 |

EPO FORM P0459

For more details about this annex : see Official Journal of the European Patent Office, No. 12/82